(19) Europäisches Patentamt
European Patent Office
Office européen des brevets

(11) **EP 3 018 480 A1**

(12) **EUROPEAN PATENT APPLICATION**
published in accordance with Art. 153(4) EPC

(43) Date of publication:
**11.05.2016 Bulletin 2016/19**

(21) Application number: **14819349.3**

(22) Date of filing: **15.04.2014**

(51) Int Cl.:
*G01N 33/72* (2006.01)    *G01J 3/42* (2006.01)
*G01J 3/46* (2006.01)    *G01N 33/50* (2006.01)
*G01N 33/52* (2006.01)    *G01N 33/68* (2006.01)

(86) International application number:
**PCT/JP2014/060675**

(87) International publication number:
**WO 2015/001831 (08.01.2015 Gazette 2015/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.07.2013  JP 2013138848**

(71) Applicant: **Olympus Corporation**
**Shibuya-ku**
**Tokyo 151-0072 (JP)**

(72) Inventors:
• **TAKEICHI, Rie**
**Tokyo 151-0072 (JP)**
• **MORIYA, Nao**
**Tokyo 151-0072 (JP)**

• **SANUKI, Hiromi**
**Tokyo 151-0072 (JP)**
• **NAKATA, Mari**
**Tokyo 151-0072 (JP)**
• **KIYOHARA, Masanobu**
**Tokyo 151-0072 (JP)**
• **TAKEYAMA, Tetsuhide**
**Tokyo 151-0072 (JP)**
• **NAGAOKA, Tomonori**
**Tokyo 151-0072 (JP)**

(74) Representative: **Gunzelmann, Rainer**
**Wuesthoff & Wuesthoff**
**Patentanwälte PartG mbB**
**Schweigerstraße 2**
**81541 München (DE)**

(54) **METHOD FOR SELECTING DUODENAL FLUID SAMPLE FOR DETECTING PANCREATIC DISEASE MARKER, AND METHOD FOR DETECTING PANCREATIC DISEASE MARKER**

(57) Provided are a method for exclusively selecting a duodenal fluid sample having a high possibility of containing pancreatic fluid and favorable sample suitability for subjecting to detection of a pancreatic disease marker by evaluating the quality of the sample prior to detecting the pancreatic disease marker, and a method for detecting a pancreatic disease marker using a duodenal fluid sample selected according to that method. Namely, a method is provided for selecting a duodenal fluid sample for detecting a pancreatic disease marker, comprising: (a1) a step of comparing the color depth of a duodenal fluid sample with a prescribed standard color, and (b1) a step of determining that a duodenal fluid sample is subjected to a test for a pancreatic disease marker if the color depth thereof is equal to or higher than the standard color, but that the duodenal fluid sample is not subjected to a test for a pancreatic disease marker if the color depth thereof is lower than the standard color.

**Description**

TECHNICAL FIELD

The present invention relates to a method for selecting a duodenal fluid sample suitable for detecting a pancreatic disease marker by evaluating its suitability as a sample that allows the obtaining of highly reliable results in the case of subjecting a duodenal fluid sample to a pancreatic disease marker test, and a method for detecting a pancreatic disease marker using a duodenal fluid sample selected according to that method.

The present application claims priority on the basis of Japanese Patent Application No. 2013-138848, filed in Japan on July 2, 2013, the contents of which are incorporated herein by reference.

BACKGROUND ART

Pancreatic fluid (fluid discharged from the pancreatic duct) is an important biological sample for determining the status of the pancreas, and is used in cytology, measurement of bicarbonate concentration, bacterial testing and testing for pancreatic diseases such as testing for markers composed of proteins or nucleic acids and the like. In particular, analyzing cells and various types of biological components contained in pancreatic fluid can be expected to lead to early detection of pancreatic cancer, which in addition to being difficult to detect at an early stage, also has a poor prognosis.

Pancreatic fluid is typically collected by transendoscopically inserting a catheter into the pancreatic duct of the pancreas. However, this method is associated with problems, such as being highly invasive to the patient and requiring the acquisition of a high level of skill by the physician. Therefore, a method has been reported in which testing for pancreatic disease is carried out using duodenal fluid instead of pancreatic fluid collected from the pancreatic duct (see, for example, Patent Document 1). Since pancreatic fluid is discharged into the duodenum from the pancreas, testing for pancreatic disease can be carried out by detecting pancreatic fluid components present in duodenal fluid. Duodenal fluid does not require an approach to the pancreatic duct in the collection step, and can be collected by inserting an endoscope into the duodenum and aspirating duodenal fluid at the site. Namely, collection of duodenal fluid can be carried out using a lowly invasive and simple technique in comparison with the collection of pancreatic fluid from the pancreatic duct.

In addition to pancreatic fluid discharged from the pancreas, duodenal fluid can also contain bile, which is discharged via the gall bladder after having been formed in the liver, as well as mucous, which is secreted in the duodenum. In other words, duodenal fluid is a fluid that consists of a mixture of pancreatic fluid, bile and duodenal fluid, and the content of each component varies. Consequently, it is unknown as to whether or not pancreatic fluid, which is thought to have the most important information in terms of testing for pancreatic disease, will always be contained in duodenal fluid collected from a subject. Even in the case of having collected a plurality of fractions from the same person, since there are deviations in the distribution of pancreatic fluid in these fractions, there is no guarantee that pancreatic fluid will necessarily be contained in the collected duodenal fluid. In actuality, although research has been conducted that attempted to distinguish between healthy subjects and chronic pancreatitis patients by measuring the level of carcinoembryonic antigen (CEA) in pancreatic fluid obtained in a test consisting of collecting pancreatic fluid, discharged 30 minutes and 45 minutes after intravenously injecting pancreatic fluid and bile secretion stimulators (secretin and CCK), from a tube inserted into the duodenum (see Non-Patent Document 1), this method is expected to have poor detection accuracy when the number of cases is increased due to the small difference in CEA concentration ranges between the healthy subject group and pancreatitis patient group.

[Prior Art Documents]

[Patent Documents]

[Patent Document 1] International Publication No. WO 2013/038981

[Non-Patent Documents]

[Non-Patent Document 1] Rolny, et al., Scandinavian Journal of Gastroenterology, 1977, Vol. 12, pp. 759-763

DISCLOSURE OF THE INVENTION

[Problems to be Solved by the Invention]

In the case of carrying out a test for a pancreatic disease marker using a duodenal fluid sample, reliable test results cannot be obtained unless pancreatic fluid is contained in the duodenal fluid sample subjected to testing. Consequently, it is important to select a duodenal fluid sample that has a high possibility of containing pancreatic fluid and is suitable as a sample in terms of enhancing testing efficiency by suppressing decreases in test accuracy caused by false negatives.

Namely, an object of the present invention is to provide a method for exclusively selecting a duodenal fluid sample having a high possibility of containing pancreatic fluid and favorable sample suitability for subjecting to detection of a pancreatic disease marker by evaluating the quality of the sample prior to detecting the pancreatic disease marker, and a method for detecting a pancreatic disease marker using a duodenal fluid sample selected according to that method.

[Means for Solving the Problems]

As a result of conducting extensive studies to achieve the aforementioned objects, the inventors of the present invention found that there are many cases in which duodenal fluid having a dark color contains more pancreatic fluid components than duodenal fluid that is nearly colorless and clear, and that duodenal fluid having a dark color for which the pH thereof is neutral to alkaline, or duodenal fluid in which the presence of P-amylase has been confirmed, is suitable as a sample for detection of a pancreatic disease marker, thereby leading to completion of the present invention.

Namely, the method for selecting a duodenal fluid sample for detecting a pancreatic disease marker and the method for detecting a pancreatic disease marker according to the present invention are as indicated in [1] to [18] below.

[1] A method for selecting a duodenal fluid sample for detecting a pancreatic disease marker, comprising:

> (a1) a step of comparing the color depth of a duodenal fluid sample with a prescribed standard color, and
> (b1) a step of determining that a duodenal fluid sample is subjected to a test for a pancreatic disease marker if the color depth thereof is equal to or higher than the standard color, but that the duodenal fluid sample is not subjected to a test for a pancreatic disease marker if the color depth thereof is lower than the standard color.

[2] A method for selecting a duodenal fluid sample for detecting a pancreatic disease marker, comprising:

> (a1) a step of comparing the color depth of a duodenal fluid sample with a prescribed standard color,
> (a2) a step of comparing the pH of the duodenal fluid sample with a prescribed standard value, and
> (b2) a step of determining that a duodenal fluid sample is subjected to a test for a pancreatic disease marker if the color depth thereof is equal to or higher than the standard color and the pH thereof is equal to or higher than the standard value, but that the duodenal fluid sample is not subjected to a test for a pancreatic disease marker if the color depth thereof is lower than the standard color or the pH thereof is lower than the standard value.

[3] The method for selecting a duodenal fluid sample for detecting a pancreatic disease marker described in [2] above, wherein the step (a2) is carried out only on a duodenal fluid sample for which the color depth of the duodenal fluid sample is equal to or higher than the standard color as determined in the step (a1).

[4] A method for selecting a duodenal fluid sample for detecting a pancreatic disease marker, comprising:

> (a1) a step of comparing the color depth of a duodenal fluid sample with a prescribed standard color,
> (a2) a step of comparing the pH of the duodenal fluid sample with a prescribed standard value,
> (a3) a step of comparing the P-amylase concentration in the duodenal fluid sample with a prescribed standard concentration, and
> (b3) a step of determining that a duodenal fluid sample is subjected to a test for a pancreatic disease marker if the color depth thereof is equal to or higher than the standard color, the pH thereof is equal to or higher than the standard value and the P-amylase concentration thereof is equal to or higher than the standard concentration, but that the duodenal fluid sample is not subjected to a test for a pancreatic disease marker if the color depth thereof is lower than the standard color, the pH thereof is lower than the standard value, or the P-amylase concentration thereof is lower than the standard concentration.

[5] The method for selecting a duodenal fluid sample for detecting a pancreatic disease marker described in [4] above, wherein the step (a2) or the step (a3) is carried out only on a duodenal fluid sample that has been determined to be suitable for testing for a pancreatic disease marker in the step (a1).

[6] A method for selecting a duodenal fluid sample for detecting a pancreatic disease marker, comprising:

> (a1) a step of comparing the color depth of a duodenal fluid sample with a prescribed standard color,
> (a3) a step of comparing the P-amylase concentration in the duodenal fluid sample with a prescribed standard concentration, and

(b4) a step of determining that a duodenal fluid sample is subjected to a test for a pancreatic disease marker if the color depth thereof is equal to or higher than the standard color and the P-amylase concentration thereof is equal to or higher than the standard concentration, but that the duodenal fluid sample is not subjected to a test for a pancreatic disease marker if the color depth thereof is lower than the standard color or the P-amylase concentration thereof is lower than the standard concentration.

[7] The method for selecting a duodenal fluid sample for detecting a pancreatic disease marker described in [6] above, wherein the step (a3) is carried out only on a duodenal fluid sample that has been determined to be suitable for testing for a pancreatic disease marker in the step (a1).

[8] The method for selecting a duodenal fluid sample for detecting a pancreatic disease marker described in any of [1] to [7] above, wherein the standard color is the color of a 0.005 mg/mL aqueous bilirubin solution or a 0.04 mg/mL aqueous bilirubin solution.

[9] The method for selecting a duodenal fluid sample for detecting a pancreatic disease marker described in any of [1] to [8] above, wherein the comparing of the color depth of a duodenal fluid sample is carried out visually.

[10] The method for selecting a duodenal fluid sample for detecting a pancreatic disease marker described in any of [1] to [8] above, wherein comparison of the color depth of a duodenal fluid sample is carried out based on spectral information of the duodenal fluid sample.

[11] The method for selecting a duodenal fluid sample for detecting a pancreatic disease marker described in any of [1] to [8] above, wherein the chromaticity point interval between the duodenal fluid sample and water, and the chromaticity point interval between the standard color and water are compared on a chromaticity diagram of the CIE XYZ color system or CIE L*a*b* color system in the step (a1), and

in the step (b1), (b2), (b3) or (b4), the duodenal fluid sample is determined to be subjected to a test for a pancreatic disease marker if the chromaticity point interval between the duodenal fluid sample and water is equal to or greater than the chromaticity point interval between the standard color and water, but not subjected to a test for a pancreatic disease marker if the chromaticity point interval between the duodenal fluid sample and water is less than the chromaticity point interval between the standard color and water.

[12] The method for selecting a duodenal fluid sample for detecting a pancreatic disease marker described in any of [1] to [8] above, wherein the comparing of color depth of a duodenal fluid sample is carried out based on optical absorbance at a prescribed wavelength within the range of 350 nm to 540 nm.

[13] The method for selecting a duodenal fluid sample for detecting a pancreatic disease marker described in any of [1] to [8] above, wherein the comparing of color depth of a duodenal fluid sample is carried out based on optical absorbance at a prescribed wavelength within the range of 400 nm to 460 nm.

[14] The method for selecting a duodenal fluid sample for detecting a pancreatic disease marker described in any of [1] to [5] or [8] to [13] above, wherein the prescribed standard value of the pH of the duodenal fluid sample is 7.5 or higher.

[15] The method for selecting a duodenal fluid sample for detecting a pancreatic disease marker described in any of [4] to [14] above, wherein the prescribed standard concentration of the P-amylase concentration in the duodenal fluid sample is 5000 U/L or more.

[16] A method for detecting a pancreatic disease marker, comprising:

a step of detecting a pancreatic disease marker in a duodenal fluid sample; wherein,
the duodenal fluid sample is selected according to the method for selecting a duodenal fluid sample for detecting a pancreatic disease marker described in any of [1] to [15] above.

[17] A method for detecting a pancreatic disease marker, comprising:

(a1) a step of comparing the color depth of a duodenal fluid sample with a prescribed standard color,
(b1) a step of determining that the duodenal fluid sample is subjected to a test for a pancreatic disease marker if the color depth is equal to or higher than the standard color, but that the duodenal fluid sample is not subjected to a test for a pancreatic disease marker if the color depth is lower than the standard color, and
(c1) a step of detecting a pancreatic disease marker in the duodenal fluid sample that has been determined to be subjected to a test for a pancreatic disease marker in the step (b1).

[18] The method for detecting a pancreatic disease marker described in [16] or [17] above, wherein the pancreatic disease marker is CEA.

[Effects of the Invention]

According to the method for selecting a duodenal fluid sample for detecting a pancreatic disease marker according to the present invention, a suitable sample can be easily selected as a sample to be subjected to a test for a pancreatic cancer marker by evaluating sample suitability of the duodenal fluid sample. Consequently, as a result of subjecting only a duodenal fluid sample, which has been selected to be subjected to a test for a pancreatic disease marker according to the aforementioned selection method, to a test for a pancreatic disease marker, but not subjecting a sample having low sample suitability to that test, test performance can be improved by inhibiting wasteful testing and reducing the number of pancreatic diseases that are failed to be detected (false negatives).

BRIEF DESCRIPTION OF THE DRAWINGS

FIG. 1 is a schematic diagram of one aspect of a flow chart in which, among the methods for detecting a marker according to the present invention, only liquid color is used as an indicator and a color comparison is carried out using spectral information.
FIG. 2 is a schematic diagram of one aspect of a flow chart in which, among the methods for detecting a marker according to the present invention, liquid color, pH and P-amylase concentration are used as indicators, and after having carried out a color comparison using spectral information, a comparison of pH is carried out followed by carrying out a comparison of P-amylase concentration.
FIG. 3 is a schematic diagram of one aspect of a flow chart in which, among the methods for detecting a marker according to the present invention, liquid color, pH and P-amylase concentration are used as indicators, and after carrying out a color comparison using spectral information, a comparison of P-amylase concentration is carried out followed by carrying out a comparison of pH.
FIG. 4 is a graph indicating absorption spectrum data of various duodenal fluid samples in Example 1.

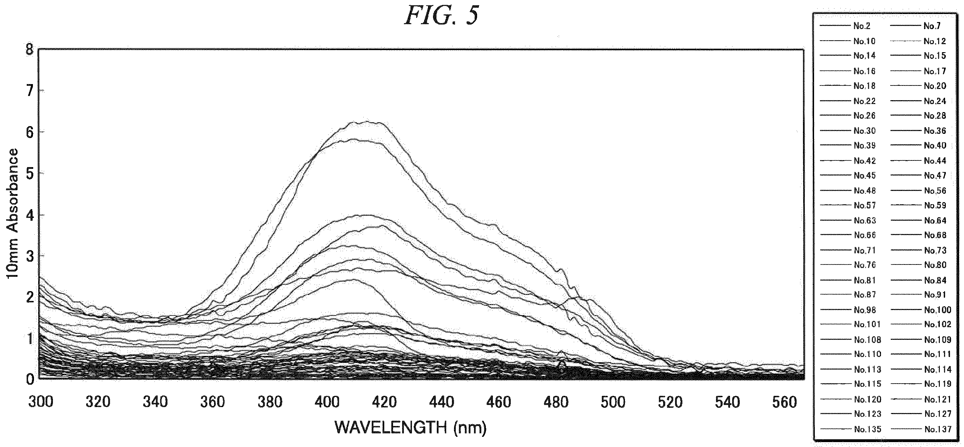

FIG. 5 is a graph indicating an enlarged view of the vicinity of the absorption peak (300-56nm) of FIG. 4.

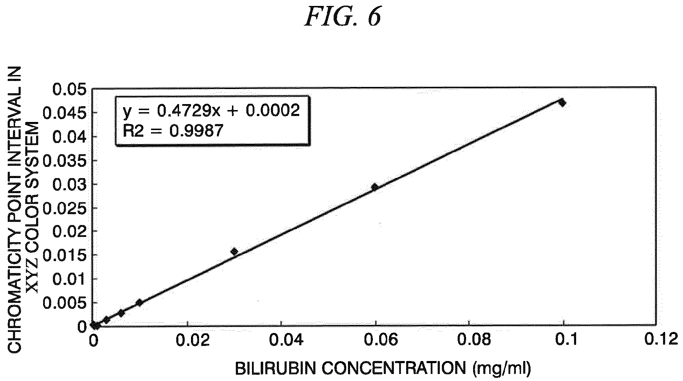

FIG. 6 is a graph indicating the results of calculating the chromaticity point intervals between the chromaticity points of aqueous bilirubin solutions having various bilirubin concentrations and the chromaticity point of water on a chromaticity diagram of the CIE XYZ color system in Example 1.

BEST MODE FOR CARRYING OUT THE INVENTION

As referred to in the present invention and description of the present application, a pancreatic disease marker constitutes various types of biological molecules, such as proteins, nucleic acids, lipids or cells, contained in pancreatic fluid, and are biological molecules for which concentration in pancreatic fluid increases significantly in patients suffering from a pancreatic disease in comparison with patients not suffering from a pancreatic disease. Furthermore, patients not suffering from a pancreatic disease include patients suffering from a disease other than a pancreatic disease. Examples of pancreatic diseases include intraductal papillary mucinous neoplasm (IPMN), mucinous cystic neoplasm (MCN), serous cystic neoplasm (SCN), neuroendocrine tumor (NET), chronic pancreatitis (CP) and acute pancreatitis.
In addition, a duodenal fluid sample that is "suitable for testing for a pancreatic disease marker" refers to a duodenal fluid sample that has a high possibility of containing pancreatic fluid and makes it possible to expect highly reliable test results with low possibilities of false negatives and false positives as a result of using that sample, while a duodenal fluid sample that is "not suitable for testing for a pancreatic disease marker" refers to a duodenal fluid sample that has a high possibility of not containing pancreatic fluid, and results in a high risk of false negatives or false positives as a result of using that sample.
Pancreatic fluid and mucous secreted in the duodenum are typically clear, while bile exhibits a yellow color due to containing yellow substances such as bilirubin. Consequently, duodenal fluid can have various color variations since it is a body fluid in which these three components are mixed at various ratios. Duodenal fluid exhibits a wide range of color ranging from light yellow to deep yellow (brownish yellow) due to the effects of yellow substances derived from bile in particular.

<Method for Selecting Duodenal Fluid Sample for Detecting Pancreatic Disease Marker>

The method for selecting a duodenal fluid sample for detecting a pancreatic disease marker according to the present invention (to also be referred to as the "sample selection method according to the present invention") is characterized in that, only a duodenal fluid sample for which the liquid color depth thereof is equal to or higher than a prescribed standard color is selected as a sample subjected to a test for a pancreatic disease marker. Namely, the duodenal fluid sample is determined to be subjected to a test for a pancreatic disease marker in the case the color depth of the duodenal fluid sample is equal to or higher than the standard color, while the duodenal fluid sample is determined to not be subjected to a test for a pancreatic disease marker in the case the color depth of the duodenal fluid sample is lower than the standard color. As indicated in Example 1 and other examples to be subsequently described, duodenal fluid having a dark color tends to have higher sensitivity for detecting a pancreatic disease marker than that having a light color, and duodenal fluid having an excessively light color has a high possibility of not containing an adequate amount of pancreatic fluid components. In other words, in the case of carrying out a test for a pancreatic disease marker on a duodenal fluid sample having excessively light color, even if the subject from whom the duodenal fluid sample was collected is suffering from a pancreatic disease, there is a high possibility of the test being negative. Therefore, by selecting a duodenal fluid sample for which liquid color depth is equal to or higher than a standard color, namely a duodenal fluid sample that can be expected to contain an adequate amount of pancreatic fluid components, by evaluating the liquid color of the duodenal fluid sample prior to subjecting to a test for a pancreatic disease marker, the frequency of false negatives can be decreased, test performance can be improved, and testing can be made more efficient.

A dark color for duodenal fluid means that it contains large amounts of bile-derived components. Although the reason why duodenal fluid containing large amounts of bile components is suitable for detection of a pancreatic disease marker is unclear, since both bile and pancreatic fluid are discharged into the duodenum from the papillary region, in the case bile is discharged into the duodenum, this is presumed to frequently be due to the papillary region being open, thereby enabling bile to be discharged into the duodenum in the same manner as pancreatic fluid. On the other hand, in the case duodenal fluid is clear, this is presumed to be due to the papillary region being obstructed, thereby preventing the discharge of both bile and pancreatic fluid. As indicated in Reference Example 1 to be subsequently described, although there was a large number of specimens that yielded false negative results among stage T4 pancreatic cancer patients in the case of testing for CEA concentration on all samples without selecting suitable duodenal fluid samples as in the prior art, this is thought to indicate the possibility of constriction of not only the pancreatic duct but also the bile duct caused by the invasion of cancer as a result of both the pancreatic duct and bile duct having been constricted due to the invasion of cancer, and is therefore presumed to be due to the duodenal fluid samples subjected to testing only containing mucous secreted from the duodenum. Furthermore, being able to use a yellow substance in the form of bile-derived components as an indicator as to whether or not pancreatic fluid components are contained in a duodenal fluid sample is a finding that was discovered for the first time by the inventors of the present invention.

The standard color for comparing color depth of a duodenal fluid sample can be suitably set so that an adequate amount of bile-derived yellow substance is contained in consideration of such factors as the type of method used to detect a pancreatic disease marker to which the duodenal fluid sample is subjected or the target test accuracy (in terms of sensitivity and specificity). In the present invention, the color of a 0.005 mg/mL aqueous bilirubin solution or 0.04 mg/mL aqueous bilirubin solution is preferably used for the standard color. Furthermore, the color of an aqueous bilirubin solution becomes darker as the bilirubin concentration becomes higher. If the color depth of a duodenal fluid sample is equal to or higher than a 0.005 mg/mL aqueous bilirubin solution, it can be expected to contain an adequate amount of pancreatic fluid components, and the sensitivity of a test for a pancreatic disease marker can be improved. Moreover, if the color depth of a duodenal fluid sample is equal to or higher than the color of a 0.04 mg/mL aqueous bilirubin solution, the frequency of false negatives in a test for a pancreatic disease marker can be reduced considerably, thereby contributing to improvement of test accuracy and greater test efficiency.

In addition, duodenal fluid may also contain gastric fluid. Since gastric fluid is highly acidic and contains pepsin and other digestive enzymes, contamination by gastric fluid has the risk of causing decomposition or degeneration of pancreatic fluid present in duodenal fluid. In addition, in the case a pancreatic disease marker targeted for detection in a test to which a duodenal fluid sample is subjected is also contained in gastric fluid, there is the problem of it being impossible to distinguish between whether the marker present in the duodenal fluid sample is derived from pancreatic fluid or gastric fluid. Moreover, a high ratio of contaminating gastric fluid indicates the risk of a low content of pancreatic fluid components. Consequently, a duodenal fluid sample having little contamination by gastric fluid can be said to be more suitable for use as a sample for detecting a pancreatic disease marker than a sample having a high degree of contamination by gastric fluid.

The pH of duodenal fluid becomes lower due to the presence of gastric fluid. Therefore, the degree of contamination of a duodenal fluid sample by gastric fluid can be estimated by using the pH of the sample as an indicator. By selecting a duodenal fluid sample for which the pH thereof is equal to or higher than a prescribed standard value, namely a duodenal fluid sample of high quality in which the amount of gastric fluid present is sufficiently low and pancreatic fluid components are not impaired to a significant extent by gastric fluid; and by measuring the pH of the duodenal fluid sample prior to subjecting to a test for a pancreatic disease marker, the frequency of false negatives can be decreased, test performance can be improved, and testing can be made more efficient.

On the other hand, $\alpha$-amylase consists of an S-isozyme and P-isozyme. Although the S-isozyme is present in the salivary gland as well as the ovaries, lungs, liver and small intestine, the P-isozyme is specific to the pancreas. In other words, if P-amylase is present in duodenal fluid, the duodenal fluid can be expected to contain pancreatic fluid.

Conversely, in the case P-amylase is not present in duodenal fluid or only present in a trace amount, there is a high possibility that the duodenal fluid does not contain an adequate amount of pancreatic fluid. Therefore, by selecting a duodenal fluid sample for which the concentration of P-amylase is equal to or higher than a prescribed standard concentration by measuring P-amylase concentration of the duodenal fluid sample prior to subjecting to a test for a pancreatic disease marker, the frequency of false negatives can be decreased, test performance can be improved, and testing can be made more efficient.

In summary of the above, a duodenal fluid sample having a dark color as opposed to a light color, a low pH as opposed to a high pH, or a high P-amylase concentration as opposed to a low P-amylase concentration, has higher suitability for use in a test for a pancreatic disease marker. Therefore, in a sample selection method according to the present invention, a duodenal fluid sample is preferably selected as a sample subjected to a test for a pancreatic disease marker only when the duodenal fluid sample is suitable for testing with respect to either of indicators consisting of the color and pH of the duodenal fluid sample or the color and P-amylase concentration of the duodenal fluid sample. The color, pH and P-amylase concentration of a duodenal fluid sample may also all be used as indicators.

Namely, among the sample selection methods according to the present invention, the method for selecting a duodenal fluid sample using only color as an indicator is characterized by comprising the following steps (a1) and (b1):

(a1) a step of comparing the color depth of a duodenal fluid sample with a prescribed standard color, and
(b1) a step of determining that a duodenal fluid sample is subjected to a test for a pancreatic disease marker if the color depth thereof is equal to or higher than the standard color, but that the duodenal fluid sample is not subjected to a test for a pancreatic disease marker if the color depth thereof is lower than the standard color.

In addition, among the sample selection methods according to the present invention, the method for selecting a duodenal fluid sample using the color and pH thereof is characterized by comprising the following steps (a1), (a2) and (b2). Selecting a duodenal fluid sample based on both color and pH makes it possible to more efficiently select a duodenal fluid sample that is highly suitable for testing for a pancreatic disease marker:

(a1) a step of comparing the color depth of a duodenal fluid sample with a prescribed standard color,
(a2) a step of comparing the pH of the duodenal fluid sample with a prescribed standard value, and
(b2) a step of determining that a duodenal fluid sample is subjected to a test for a pancreatic disease marker if the color depth thereof is equal to or higher than the standard color and the pH thereof is equal to or higher than the standard value, but that the duodenal fluid sample is not subjected to a test for a pancreatic disease marker if the color depth thereof is lower than the standard color or the pH thereof is lower than the standard value.

In this method, step (a1) and step (a2) may be carried out in any order.

In this method, although step (a1) and step (a2) may be carried out on all duodenal fluid samples, it is preferable to carry out step (a1) followed by only carrying out step (a2) on those duodenal fluid samples for which color depth is equal to or higher than the standard color as determined in step (a1) (namely, only those duodenal fluid samples that have been judged to be suitable). As a result, the number of duodenal fluid samples investigated for pH in step (a2) can be limited, thereby making it possible to reduce the amount of labor spent on the entire method. Furthermore, step (a1) may be carried out only on those duodenal fluid samples for which the pH thereof is equal to or higher than a standard value as determined in step (a2) after having first carried out step (a2).

In addition, among the sample selection methods according to the present invention, the method for selecting a duodenal fluid sample using the color, pH and P-amylase concentration thereof is characterized by comprising the aforementioned step (a1) and the following steps (a2), (b2) and (b3). Selecting a duodenal fluid sample based on all of color, pH and P-amylase concentration makes it possible to more efficiently select a duodenal fluid sample that is highly suitable for testing for a pancreatic disease marker:

(a2) a step of comparing the pH of the duodenal fluid sample with a prescribed standard value,
(a3) a step of comparing the P-amylase concentration in the duodenal fluid sample with a prescribed standard concentration, and
(b3) a step of determining that a duodenal fluid sample is subjected to a test for a pancreatic disease marker if the color depth thereof is equal to or higher than the standard color, the pH thereof is equal to or higher than the standard value and the P-amylase concentration thereof is equal to or higher than the standard concentration, but that the duodenal fluid sample is not subjected to a test for a pancreatic disease marker if the color depth thereof is lower than the standard color, the pH thereof is lower than the standard value, or the P-amylase concentration thereof is lower than the standard concentration.

In this method, step (a1), step (a2) and step (a3) may be carried out in any order.

In this method, it is preferable to carry out step (a1) followed by carrying out step (a2) and step (a3) only on those duodenal fluid samples for which color depth is equal to or higher than the standard color as determined in step (a1). In addition, it is more preferable to carry out step (a1) followed by carrying out step (a2) only on those duodenal fluid samples for which color depth is equal to higher than the standard color as determined in that step, and carrying out step (a3) only on those duodenal fluid samples for which pH is equal to or higher than the standard value as determined in that step (a2) (namely, duodenal fluid samples judged to be suitable for testing); or carry out step (a1) followed by carrying out step (a3) only on those duodenal fluid samples for which color depth is equal to or higher than the standard color as determined in step (a1), and carrying out step (a2) only on those duodenal fluid samples for which P-amylase concentration is equal to or higher than the standard concentration as determined in step (a3) (namely, duodenal fluid samples judged to be suitable for testing). As a result of carrying out the remaining steps only on duodenal fluid samples that have been judged to be suitable for testing in the previous step in this manner, the amount of labor spent on the entire method can be reduced. Furthermore, step (a1) may be carried out after carrying out step (a2) only on those duodenal fluid samples that have been judged to be suitable for testing for a pancreatic disease marker in step (a2), or step (a1) and step (a2) may be carried out after carrying out step (a3) only on those duodenal fluid samples that have been judged to be suitable for testing for a pancreatic disease marker in step (a3).

Among the sample selection methods according to the present invention, the method for selecting a duodenal fluid sample using the color and P-amylase concentration thereof is characterized by comprising the aforementioned steps (a1) and (a3) and the following step (b4). Selecting a duodenal fluid sample based on both color and P-amylase concentration makes it possible to more efficiently select a duodenal fluid sample that is highly suitable for testing for a pancreatic disease marker:

(b4) a step of determining that a duodenal fluid sample is subjected to a test for a pancreatic disease marker if the color depth thereof is equal to or higher than the standard color and the P-amylase concentration thereof is equal to or higher than the standard concentration, but that the duodenal fluid sample is not subjected to a test for a pancreatic disease marker if the color depth thereof is lower than the standard color or the P-amylase concentration thereof is lower than the standard concentration.

In this method, step (a1) and step (a3) may be carried out in any order. In addition, similar to the previously described methods, it is preferable in this method as well to carry out step (a1) followed by carrying out step (a3) only on those duodenal fluid samples for which the color depth thereof is equal to or higher than the standard color as determined in step (a1).Step (a3) may also be carried out followed by carrying out step (a1) only on those duodenal fluid samples for which P-amylase concentration is equal to or higher than the standard concentration as determined in step (a3).

Although the duodenal fluid sample targeted for selection in the sample selection method according to the present invention may be a duodenal fluid sample collected from a location in the intestinal tract of the duodenum, it is preferably duodenal fluid present in the second portion or third portion of the duodenum. This is because the first portion of the duodenum is located at a site connected directly to the pyloric region of the stomach resulting in a high possibility of contamination by gastric fluid, and may make collection difficult since it is comparatively difficult to immobilize the endoscope used to collect the fluid.

Furthermore, duodenal fluid can be collected in accordance with routine methods. For example, duodenal fluid can be collected with an aspiration means such as a syringe or vacuum pump connected to an endoscopic catheter. More specifically, after inserting an endoscope through the oral cavity into the duodenum, duodenal fluid present in the second and third portions is collected by aspirating using a catheter inserted by passing through a forceps channel. For example, duodenal fluid that has accumulated in the intestinal tract of the duodenum may be collected when undergoing an endoscopic examination of the stomach and duodenum (upper endoscopy) during a so-called gastroscopy.

The color of duodenal fluid samples may differ even if collected on the same day from the same subject. The reason for this is thought to be differences in the sites where the duodenal fluid samples are collected and differences in the times at which they are collected. Therefore, in the case of a plurality of duodenal fluid samples collected from the same subject, the sample having the darkest color is compared with a prescribed standard color. Furthermore, in the case of a plurality of duodenal fluid samples collected from the same subject, step (a2) and step (a3) are carried out on a duodenal fluid sample for which the color depth thereof is equal to or higher than the standard color when compared with that standard color (namely, the duodenal fluid sample subjected to step (a1)).

Since duodenal fluid normally contains a diverse range of enzymes, there is the risk of decomposition and degeneration of components in a duodenal fluid sample depending on the state in which the sample is stored. In addition, colored components such as bilirubin have the possibility of being decomposed by light. Consequently, in the case time is required from the time a duodenal fluid sample is collected from a subject until the color depth thereof is compared with the standard color, the duodenal fluid sample is preferably stored refrigerated or frozen while blocking from light as necessary.

In addition, various types of additives or buffers and the like may be added to a duodenal fluid sample collected from a subject prior to comparing the color depth thereof with the standard color provided that addition does not change the color depth of the fluid. Examples of additives include reagents for inhibiting decomposition or degeneration of components in the duodenal fluid sample, such as surfactants, protease inhibitors or nuclease inhibitors. These additives and the like may be added to a container containing duodenal fluid collected from the body, or may be placed in a container in advance followed by collecting duodenal fluid directly into the container.

Comparison of the color of a duodenal fluid sample with a standard color can be carried out visually. Carrying out this comparison visually eliminates the need for special analysis devices and procedures and enables the sample having the darkest color to be selected both easily and rapidly from a plurality of duodenal fluid samples. For example, by preliminarily preparing a color sample having the standard color attached thereto and visually comparing a duodenal fluid sample with the color sample, the duodenal fluid sample is determined to be subjected to a test for a pancreatic disease marker in the case the color of the duodenal fluid sample is equal to or darker than the standard color of the color sample, while the duodenal fluid sample is determined to not be subjected to a test for a pancreatic disease marker in the case the color of the duodenal is lighter than the standard color.

Comparison of the color of a duodenal fluid sample with a standard color can also be carried out based on spectral information of each duodenal fluid sample. Optical spectrum refers to absorbance spectrum or transmission spectrum and the like. The use of spectral information makes it possible to compare slight differences in color that are difficult to compare visually by comparing in the form of numerical values. In addition, color comparisons can be carried out objectively while also allowing comparison of a large number of samples to be carried out easily. The optical spectrum of a duodenal fluid sample can be measured using various types of devices, such as a spectrophotometer, typically used for spectral analysis of liquid samples.

Yellow substances such as bilirubin derived from bile have a peak wavelength range of the absorption spectrum thereof of 350 nm to 540 nm, and particularly 400 nm to 460 nm. Therefore, color depth can be compared with a standard color based on absorption spectrum data of the duodenal fluid specimen in this wavelength range. More specifically, the absorbance of a duodenal fluid sample and a liquid having the standard color (standard liquid such as an aqueous bilirubin solution having a concentration of 0.005 mg/mL) is measured and compared at a prescribed wavelength within a range of 350 nm to 540 nm. Color is judged to be darker the greater this optical absorbance. In other words, the duodenal fluid sample is determined to be subjected to a test for a pancreatic disease marker in the case this optical absorbance is equal to or higher than that of the standard liquid, while the duodenal fluid sample is determined to not be subjected to a test for a pancreatic disease marker in the case this optical absorbance is lower than that of the standard liquid. This optical absorbance is only required to be optical absorbance at a wavelength within a range of 350 nm to 540 nm, is preferably optical absorbance at a wavelength within a range of 400 nm to 460 nm, and is more preferably optical absorbance at a wavelength within a range of 450 nm to 460 nm.

In addition, the comparison between the color depth of a duodenal fluid sample and the depth of a standard color is also preferably carried out based on the chromaticity point interval with water in a chromaticity diagram of the CIE (Commission Internationale de l'Eclairage (International Commission on Illumination)) XYZ color system (or CIE Yxy color system) or CIE L*a*b* color system. More specifically, the absorption spectrum $[A_{10}]$ at 380 nm to 780 nm is measured for a duodenal fluid sample followed by converting this to transmission spectrum $[T_{10}]$ in accordance with the equation indicated below.

[Equation 1]

$$T_{10} = 10^{-A_{10}}$$

The color of the duodenal fluid sample is then determined from the resulting transmission spectrum in the form of a chromaticity point of a color system. Next, the chromaticity point interval on a chromaticity diagram is then calculated between this chromaticity point and the chromaticity point of water (colorless, clear solution) determined in the same manner. This chromaticity point interval with water represents "color depth", and a larger chromaticity point interval is judged to indicate a darker color, while a smaller chromaticity point interval is judged to indicate a lighter color. In the case the calculated chromaticity point interval with water is equal to or greater than the chromaticity point interval between water and the standard liquid calculated in the same manner, the duodenal fluid sample is determined to be subjected to a test for a pancreatic disease marker, while in the case the chromaticity point interval is less than the chromaticity point interval between the standard liquid and water, the duodenal fluid sample is determined to not be subjected to a test for a pancreatic disease marker.

Chromaticity of a duodenal fluid sample and standard liquid in each color system can be determined in accordance with routine methods. Furthermore, since the comparison described here is a relative comparison between a duodenal fluid sample and a standard liquid, the duodenal fluid sample and standard liquid are only required to be measured under the same conditions, while various conditions when measuring absorption spectrum or determining chromaticity in each color system (such as the light source, filter or optics) are arbitrary.

The pH of gastric fluid is in the vicinity of 2, while the pH of pancreatic fluid and bile is in the vicinity of 8. Consequently, the pH of duodenal fluid approaches the vicinity of neutral pH when gastric fluid and duodenal fluid mix in the duodenum. In the case a duodenal fluid sample exhibits an acidic pH, a large amount of gastric fluid can be predicted to be mixed into the collected duodenal fluid. Therefore, the standard value of the pH of a duodenal fluid sample is more preferably set to within the neutral to alkaline range, even more preferably set to a pH of 7.5 or higher, and may be set to a pH of 8.2 or higher. As a result of setting the standard value of pH to within these ranges, the effect of contamination by gastric fluid is reduced and a duodenal fluid sample of high quality can be selected. Furthermore, the pH of a duodenal fluid sample can be measured by suitably selecting a method from among known measurement methods such as the use of pH test paper or measuring with a pH meter.

The P-amylase concentration in a duodenal fluid sample compared in step (a3) may be the protein concentration per se of that enzyme or may be an enzymatic activity concentration (U/L). In the case of using enzymatic activity concentration, the standard concentration of P-amylase concentration in a duodenal fluid sample is preferably 5000 U/L or more and may be 10000 U/L or more. Furthermore, enzymatic activity concentration of P-amylase can be measured using a method such as immuno-inhibition (method by which amylase activity is measured using a labeled substrate in the presence of a monoclonal antibody that specifically inhibits S-amylase only) typically used in clinical laboratory testing.

<Method for Detecting Pancreatic Disease Marker>

The method for detecting a pancreatic disease marker according to the present invention (to also be referred to as the marker detection method according to the present invention) is characterized by exclusively subjecting a duodenal fluid sample selected according to the sample selection method according to the present invention to detection of a pancreatic disease marker. By selecting a duodenal fluid sample having a high possibility of containing an adequate amount of pancreatic fluid in accordance with the sample selection method according to the present invention and detecting a pancreatic disease marker in the selected duodenal fluid sample, detection sensitivity of the pancreatic disease marker is high and highly reliable detection results can be obtained.

A duodenal fluid sample selected according to a sample selection method according to the present invention may be used to detect a pancreatic disease marker (pancreatic disease marker measurement test) immediately after selection, or may be used to detect a pancreatic disease marker after storing for a prescribed amount of time after selection. A duodenal fluid specimen following selection may be stored refrigerated or frozen, may be stored at room temperature, or may be stored in the form of a freeze-dried powder by undergoing freeze-drying treatment. In addition, it may be stored after adding various additives thereto. Examples of such additives include surfactants, protease inhibitors, nuclease inhibitors, pH adjusters and pH indicators. Moreover, a selected duodenal fluid sample may be used as is in a pancreatic disease marker measurement test, or may be used after having separated and removed cells and other solids by undergoing a centrifugal separation procedure and the like.

A pancreatic disease marker detected in the marker detection method according to the present invention is only required to be a biological molecule for which the concentration thereof in pancreatic fluid increases significantly in patients suffering from a pancreatic disease in comparison with patients not suffering from a pancreatic disease, and there are no particular limitations thereon. In the present invention, the pancreatic disease marker is preferably a biological molecule such as a glycoprotein or enzyme that is resistant to the effects of digestive enzymes contained in duodenal fluid. Examples of pancreatic disease markers detected with the marker detection method according to the present invention include glycoproteins in the form of CEA, CA19-9 (see, for example, Pancreas, 1994, Vol. 9, No. 6, pp. 741-747) and MUC-1 (KL-6) (see, for example, Japanese Unexamined Patent Application, First Publication No. 2006-308576), and enzymes in the form of MMP2 (Matrix Metalloproteinase-2) (see, for example, Pancreas, 2002, Vol. 24, No. 54, pp. 344-347) and MMP7 (Matrix Metalloproteinase-7). Other examples include substances such as S100P, NGAL and MIC-1 for which the concentration thereof increases significantly in patients suffering from a pancreatic disease.

The pancreatic disease marker detected with the marker detection method according to the present invention is particularly preferably CEA. The onset and risk of onset of diseases such as pancreatic cancer, IPMN, MCN, chronic pancreatitis or acute pancreatitis can be investigated by using the concentration of CEA in duodenal fluid as an indicator.

In the marker detection method according to the present invention, there are no particular limitations on the method used to detect a pancreatic disease marker provided it is a test performed for the purpose of detecting or quantifying a pancreatic disease marker in a sample. For example, various types of pancreatic disease markers can be detected by various types of protein analyses using ELISA, immunochromatography, two-dimensional electrophoresis, Western blotting or mass spectrometry and the like, various types of nucleic acid analyses using PCR, RT-PTR or hybridization using a probe, or cell analyses in the manner of cell counting or cytodiagnosis. In addition, a pancreatic disease marker can be detected or quantified both quickly and easily in a large number of duodenal fluid samples by using various types of analyzers such as automated biochemical analyzers.

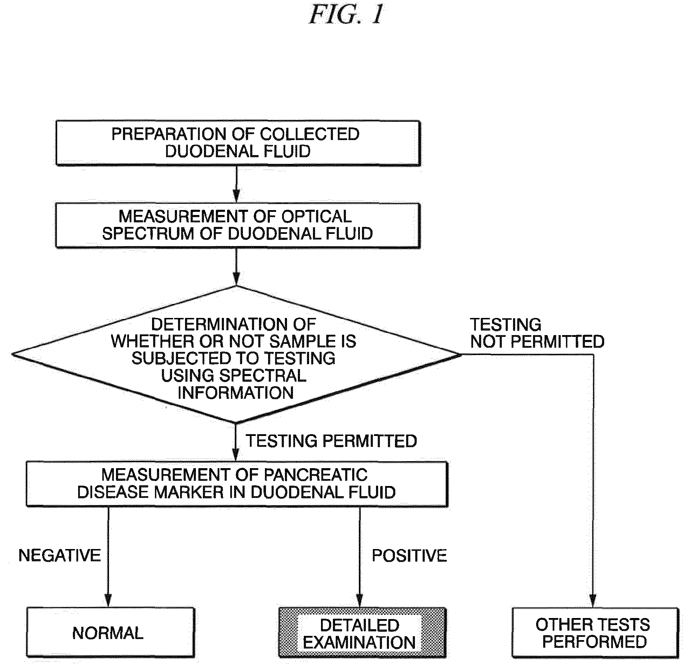

FIG. 1 indicates a schematic diagram of one aspect of a flow chart in which a color comparison is carried out using spectral information by using only liquid color as an indicator in the marker detection method according to the present invention. As was previously described, the optical spectrum of a duodenal fluid sample collected from a subject is first measured. Next, the resulting spectral information is compared with the spectral information of a standard liquid to determine whether or not the duodenal fluid sample is subjected to a test for a pancreatic disease marker. A pancreatic disease marker present in the duodenal fluid sample is then measured for the duodenal fluid sample selected to be subjected to testing (able to be tested) for a pancreatic disease marker. Testing for a pancreatic disease marker is not carried out on a duodenal fluid sample that has been determined to not be subjected to testing (unable to be tested) for a pancreatic disease marker, and other testing on that sample is examined.

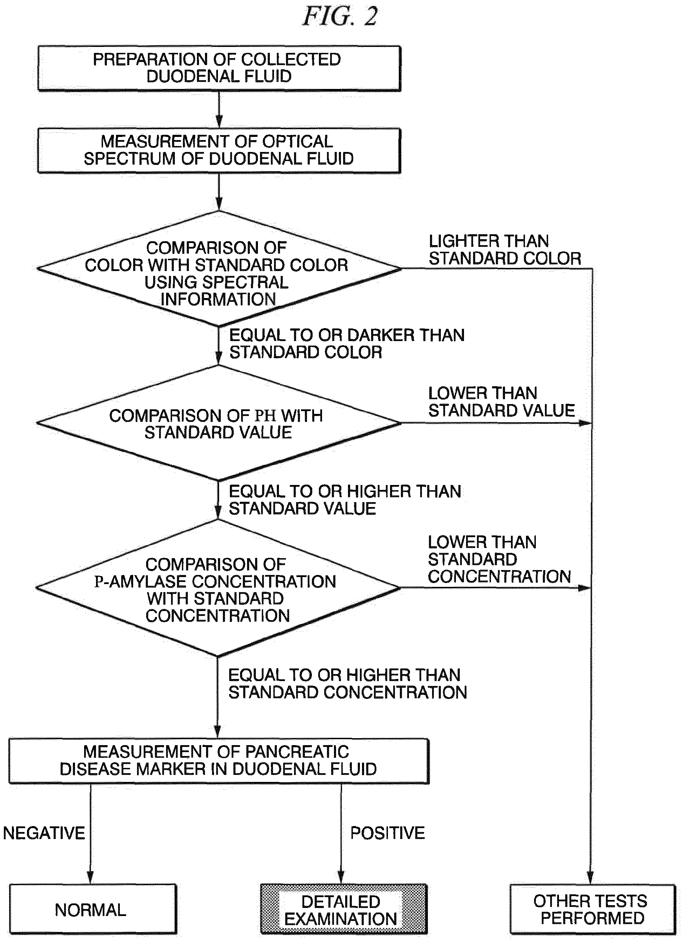

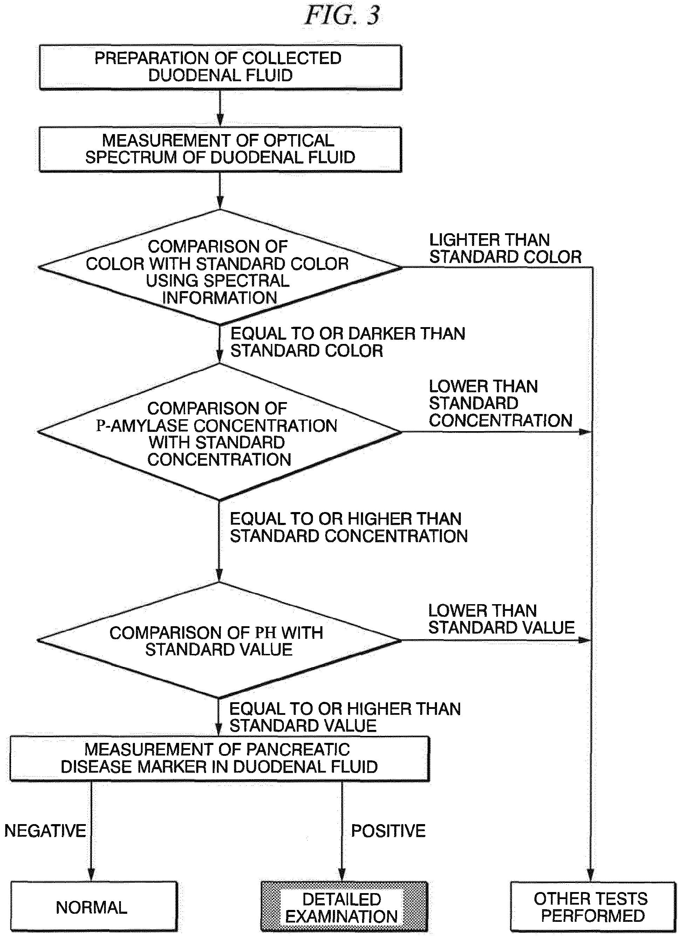

FIG. 2 indicates a schematic diagram of one aspect of a flow chart in which testing is carried out on a duodenal fluid sample selected by using liquid color, pH and P-amylase concentration as indicators, evaluating testing suitability by carrying out a color comparison using spectral information, evaluating testing suitability by comparing pH, and subsequently evaluating testing suitability according to P-amylase concentration in the marker detection method according to the present invention. In addition, FIG. 3 indicates a schematic diagram of one aspect of a flow chart in which testing is carried out on a duodenal fluid sample selected by using liquid color, pH and P-amylase concentration as indicators, evaluating testing suitability by carrying out a color comparison using spectral information, evaluating testing suitability according to P-amylase concentration, and subsequently evaluating testing suitability by comparing pH in the marker detection method according to the present invention. As was previously described, in the flow chart described in FIG. 2, the optical spectrum is first measured for a duodenal fluid sample collected from a subject. Next, the resulting spectral information is compared with the spectral information of a standard liquid. The duodenal fluid sample is judged to be unable to be tested if the color depth thereof is lower than a standard color, while the pH of the duodenal fluid sample is compared with a standard value if the color depth thereof is equal to or higher than the aforementioned standard color. The duodenal fluid sample is judged to be unable to be tested if the pH thereof is lower than the aforementioned standard value, while the P-amylase concentration of the duodenal fluid sample is compared with a standard concentration if the pH thereof is equal to or higher than the aforementioned standard value. The duodenal fluid sample is judged to be unable to be tested if the P-amylase concentration is lower than the aforementioned standard concentration, while a pancreatic disease marker in the duodenal fluid sample is measured if the P-amylase concentration thereof is equal to or higher than the aforementioned standard concentration. Testing for a pancreatic disease marker is not carried out on duodenal fluid samples that have been judged to be unable to be tested in each step, and other testing on those samples is examined. In the flow chart described in FIG. 3, it is carried out in the same manner as the flow chart described in FIG. 2 with the exception of carrying out the step for comparing P-amylase concentration after the step for comparing color, followed by carrying out the step for comparing pH.

For example, in the case of a pancreatic disease marker in the manner of CEA for which the concentration in the duodenal fluid of a patient suffering from a pancreatic disease tends to be higher than that in a healthy subject, a duodenal fluid sample can be judged to be negative in the case the measured value thereof is less than a prescribed threshold value, and can be judged to be positive in the case the measured value thereof is equal to or higher than a prescribed threshold value. In the case of a negative result, the subject from whom the duodenal fluid sample was collected has a high possibility of being a normal subject (or a subject not suffering from a pancreatic disease), while in the case of a positive result, the subject has a high possibility of suffering from a pancreatic disease. Therefore, it is useful to examine whether or not additional follow-up testing is to be carried out on the subject evaluated as positive. Follow-up testing refers to, for example, endoscopic retrograde cholangiopancreatography (ERCP), endoscopic ultrasound/fine needle aspiration (EUS-FNA), magnetic resonance cholangiopancreatography (MRCP), computed tomography (CT) and magnetic resonance imaging (MRI).

[Examples]

Although the following provides a more detailed explanation of the present invention by indicating examples thereof, the present invention is not limited to the following examples.

[Reference Example 1]

Duodenal fluid samples were collected transendoscopically from 49 pancreatic cancer cases followed by measuring the CEA concentrations in the samples. CEA concentrations were measured by ELISA using a commercially available kit (IBL International Corp.). The samples were judged to be negative or positive based on the measured CEA concentrations using a cutoff value of 126 ng/mL.

Sensitivity and the ratios of the number of positive specimens to the total number of specimens as determined from the test results are shown in Table 1 according to T classification representing the stage of localization of the primary lesion in the pancreas. Tis represents noninvasive cancer, T1 represents that having a tumor diameter of 2 cm or less that is localized in the pancreas, T2 represents that having a tumor diameter of greater than 2 cm that is localized in the pancreas, T3 represents cancer that has invaded any of the pancreatic bile duct, duodenum or peripancreatic tissue, and T4 represents cancer that has invaded any of the adjacent large vessels, extrapancreatic neural plexus and other organs. Since the T4 group clearly has extremely low detection sensitivity in comparison with Tis, T1, T2 and T3, the failure to detect T4 cases is considered to be the primary cause of the decrease in sensitivity of testing using CEA as a pancreatic cancer marker. In addition, since the ratio of false negatives (cases of pancreatic cancer having CEA concentrations below the threshold value) was 32.7% (16 of 49 cases), there was suggested to be a comparatively high probability of failing to detect these cases.

[Table 1]

| Cancer Stage (T Classification) | Sensitivity | [No. of positive specimens]/[Total no. of specimens] |
| --- | --- | --- |
| Tis, T1, T2 | 66.7% | 6/9 |
| T3 | 80.0% | 20/25 |
| T4 | 46.7% | 7/15 |

The reason for the decrease in sensitivity in stage T4 cases is thought to be due to constriction of both the pancreatic duct and bile duct preventing discharge of both pancreatic fluid and bile from the papillary region. In actuality, many of the duodenal fluid samples collected from stage T4 patients were clear in comparison with those collected from patients having other classifications. These results suggested the usefulness of carrying out testing on duodenal fluid after screening for specimens having darker color.

[Example 1]

The absorption spectra were measured for duodenal fluid samples collected transendoscopically from 49 cases of pancreatic cancer and 7 cases of benign pancreatic diseases. The absorption spectra of all specimens are shown in FIG. 2. An enlarged view of the vicinity of the absorption peak of FIG. 2 (300 nm to 56 nm) is shown in FIG. 3. The duodenal fluid samples clearly demonstrated an absorption peak wavelength range at 350 nm to 540 nm, and particularly 400 nm to 460 nm.

Moreover, the color depth of each duodenal fluid sample (specimen) was evaluated. More specifically, the absorption spectrum of each specimen at 380 nm to 780 nm was measured using a spectrophotometer at an optical path length of 10 mm ($A_{10}$). Next, the resulting absorption spectra were converted to transmission spectra ($T_{10}$) based on the following equation followed by further converting to an optical path length of 5 mm ($T_5$).

[Equation 2]

$$T_{10} = 10^{-A_{10}}$$

$$T_5 = (-\text{Log}(T_{10}, 10)/10) * 5$$

Chromaticity composed of the tristimulus values (X,Y,Z,) of the CIE XYZ color system was then determined from the transmission spectra after converting to the optical path length followed by plotting on the XY coordinates of a chromaticity diagram. The X and Y values of each specimen are shown in Tables 2 to 5.

[Equation 3]

$$x = \frac{X}{X+Y+Z} \qquad y = \frac{Y}{X+Y+Z} \qquad z = \frac{Z}{X+Y+Z} = 1-x-y$$

$$X = K \int_{380}^{780} S(\lambda)\overline{x}(\lambda)R(\lambda)d\lambda$$

$$Y = K \int_{380}^{780} S(\lambda)\overline{y}(\lambda)R(\lambda)d\lambda$$

$$Z = K \int_{380}^{780} S(\lambda)\overline{z}(\lambda)R(\lambda)d\lambda$$

$$K = \frac{100}{\int_{380}^{780} S(\lambda)\overline{y}(\lambda)d(\lambda)}$$

S($\lambda$): Spectral distribution of standard light used to display color (light source * filter * optics; here, calculated as 1 in each case)
x~($\lambda$), y~($\lambda$), z~($\lambda$): Color-matching function of 2° field in XYZ color system
R($\lambda$): Spectral reflectance factor (here, transmittance of sample)

Moreover, optical absorbance at 455 nm and CEA concentration were also measured for each specimen. The measurement results are shown in Tables 2 to 5. CEA concentrations were measured in the same manner as Reference Example 1.

[Table 2]

| Benign Disease Specimens | | | | | | |
|---|---|---|---|---|---|---|
| Specimen No. | CEA concentration (ng/mL) | XYZ color system | | Optical absorbance (455 nm) | Sensitivity | |
| | | X value | Y value | | All specimens | Selected specimens |
| B1 | 0.0 | 0.336 | 0.334 | 0.028 | - | - |
| B2 | 0.0 | 0.338 | 0.335 | 0.037 | | |
| B3 | 125.5 | 0.464 | 0.483 | 2.609 | | |
| B4 | 88.2 | 0.353 | 0.362 | 0.314 | | |
| B5 | 95.3 | 0.341 | 0.342 | 0.113 | | |
| B6 | 49.7 | 0.342 | 0.342 | 0.082 | | |
| B7 | 26.0 | 0.346 | 0.349 | 0.208 | | |

[Table 3]

| Pancreatic Cancer (Tis, T1, T2) Specimens | | | | | | |
|---|---|---|---|---|---|---|
| Specimen No. | CEA concentration (ng/mL) | XYZ color system | | Optical absorbance (455 nm) | Sensitivity | |
| | | X value | Y value | | All specimens | Selected specimens |
| M1☆# | 48.1 | 0.398 | 0.419 | 0.824 | 66.7% (6/9) | 62.5% (5/8) |
| M2# | 291.3 | 0.343 | 0.341 | 0.096 | | |
| M3☆# | 1154.0 | 0.361 | 0.374 | 0.443 | | |
| M4☆# | 1287.0 | 0.362 | 0.376 | 0.379 | | |
| M5☆# | 1720.9 | 0.400 | 0.424 | 0.851 | | |
| M6☆# | 46.5 | 0.365 | 0.375 | 0.390 | | |
| M7☆# | 53.9 | 0.385 | 0.409 | 0.731 | | |
| M8☆# | 193.6 | 0.404 | 0.432 | 1.033 | | |
| M9☆# | 260.6 | 0.368 | 0.377 | 0.380 | | |

[Table 4]

| Pancreatic Cancer (T3) Specimens | | | | | | |
|---|---|---|---|---|---|---|
| Specimen No. | CEA concentration (ng/mL) | XYZ color system | | Optical absorbance (455 nm) | Sensitivity | |
| | | X value | Y value | | All specimens | Selected specimens |
| M10☆# | 694.8 | 0.368 | 0.378 | 0.459 | | |
| M11☆# | 30.3 | 0.374 | 0.388 | 0.497 | | |
| M12☆# | 934.0 | 0.352 | 0.356 | 0.226 | | |
| M13 | 11.4 | 0.334 | 0.335 | 0.008 | | |
| M14# | 947.0 | 0.348 | 0.348 | 0.140 | | |

(continued)

| Pancreatic Cancer (T3) Specimens | | | | | | |
|---|---|---|---|---|---|---|
| Specimen No. | CEA concentration (ng/mL) | XYZ color system | | Optical absorbance (455 nm) | Sensitivity | |
| | | X value | Y value | | All specimens | Selected specimens |
| M15# | 188.1 | 0.342 | 0.341 | 0.061 | 80.0% (20/25) | 89.5% (17/19) |
| M16☆# | 101.8 | 0.355 | 0.358 | 0.241 | | |
| M17☆# | 194.0 | 0.349 | 0.354 | 0.280 | | |
| M18# | 201.0 | 0.354 | 0.357 | 0.223 | | |
| M19# | 45.1 | 0.335 | 0.338 | 0.027 | | |
| M20 | 81.8 | 0.334 | 0.335 | 0.092 | | |
| M21☆# | 162.2 | 0.352 | 0.365 | 0.392 | | |
| M22☆# | 288.8 | 0.364 | 0.369 | 0.379 | | |
| M23☆# | 738.9 | 0.452 | 0.471 | 2.151 | | |
| M24# | 230.7 | 0.339 | 0.341 | 0.085 | | |
| M25☆# | 809.7 | 0.354 | 0.365 | 0.386 | | |
| M26☆# | 670.7 | 0.436 | 0.472 | 1.828 | | |
| M27☆# | 195.7 | 0.371 | 0.382 | 0.418 | | |
| M28☆# | 1428.6 | 0.382 | 0.389 | 0.712 | | |
| M29☆# | 892.4 | 0.390 | 0.416 | 0.865 | | |
| M30☆# | 1072.1 | 0.353 | 0.367 | 0.382 | | |
| M31# | 1312.0 | 0.336 | 0.336 | 0.109 | | |
| M32☆# | 4192.9 | 0.387 | 0.397 | 0.709 | | |
| M33☆# | 329.0 | 0.477 | 0.497 | 3.789 | | |
| M34☆# | 172.6 | 0.364 | 0.370 | 0.306 | | |

[Table 5]

| Pancreatic Cancer (T4) Specimens | | | | | | |
|---|---|---|---|---|---|---|
| Specimen No. | CEA concentration (ng/mL) | XYZ color system | | Optical absorbance (455 nm) | Sensitivity | |
| | | X value | Y value | | All specimens | Selected specimens |
| M35☆# | 1741.5 | 0.478 | 0.492 | 3.406 | 46.7% (7/15) | 75.0% (6/8) |
| M36# | 296.0 | 0.337 | 0.339 | 0.137 | | |
| M37☆# | 77.4 | 0.347 | 0.353 | 0.236 | | |
| M38☆# | 1499.7 | 0.359 | 0.372 | 0.431 | | |
| M39 | 50.4 | 0.334 | 0.335 | 0.083 | | |
| M40☆# | 237.0 | 0.349 | 0.356 | 0.239 | | |
| M41☆# | 2227.0 | 0.355 | 0.358 | 0.252 | | |
| M42☆# | 2247.9 | 0.445 | 0.475 | 1.885 | | |
| M43# | 60.1 | 0.344 | 0.344 | 0.095 | | |
| M44☆# | 189.8 | 0.353 | 0.358 | 0.234 | | |
| M45# | 22.7 | 0.337 | 0.338 | 0.075 | | |
| M46# | 46.6 | 0.335 | 0.335 | 0.066 | | |
| M47☆# | 119.7 | 0.467 | 0.476 | 2.410 | | |
| M48 | 57.2 | 0.334 | 0.334 | 0.089 | | |
| M49 | 10.7 | 0.334 | 0.334 | 0.093 | | |

As shown in Table 2, the CEA concentrations of benign specimens ranged from 0.0 ng/mL to 125.5 ng/mL. Therefore, the cutoff value for CEA concentration was set at 126 ng/mL to achieve specificity of 100%. Therefore, when 49 specimens of pancreatic cancer were evaluated using this cutoff value, 33 specimens were positive and 16 specimens were negative. In other words, the percentage of true positives was 67.3% and the percentage of false negatives was 32.7%. When sensitivity at each stage of pancreatic cancer was investigated, sensitivity was 66.7% (6/9 specimens) in the pancreatic cancer (Tis,T1,T2) group, 80.0% (20/25 specimens) in the pancreatic cancer (T3) group, and 46.7% (7/15 specimens) in the pancreatic cancer (T4) group (refer to the "Sensitivity (All specimens)" column in Tables 3 to 5).

Next, after having defined the chromaticity point having an X value of 0.345 and Y value of 0.349 in the CIE XYZ color system as the standard color (Standard Color 1), specimens having an X value of 0.345 or more and a Y value of 0.349 or more in the same color system were selected as specimens having color depth equal to or higher than that of Standard Color 1. Specimens indicated with a star (☆) in the "Specimen No. "column of Tables 3 to 5 indicate specimens that were selected based on Standard Color 1. Furthermore, only specimens that were the same as the specimens selected based on the aforementioned Standard Color 1 were selected when liquid color having optical absorbance of 0.137 at 455 nm was used for the standard color for each specimen. When only these selected specimens were investigated for sensitivity, sensitivity in the pancreatic cancer (Tis,T1,T2) group was 62.5% (5/8 specimens), sensitivity in the pancreatic cancer (T3) group was 89.5% (17/19 specimens) and sensitivity in the pancreatic cancer (T4) group was 75.0% (6/8 specimens) (refer to the "Sensitivity (Selected specimens) "column in Tables 3 to 5) . In addition, the overall sensitivity for 35 specimens selected from among the 49 pancreatic cancer specimens was 80.0% (28/35 specimens), and the percentage of false negatives was 20.0% (7/35 specimens). In contrast, among the 14 specimens that were not selected among the pancreatic cancer specimens, the percentage of true positives was 35.7% (5/14 specimens) and the percentage of false negatives was 64.3% (9/14 specimens). In this manner, although sensitivity was somewhat low in the pancreatic cancer (Tis,T1,T2) group, the case of targeting only those specimens that were selected based on Standard Color 1 resulted in greater improvement of overall sensitivity, as well as sensitivity in the pancreatic cancer (T3) group and pancreatic cancer (T4) group, in comparison with the case of targeting all specimens. In the pancreatic cancer (T4) group in particular, sensitivity improved dramatically from 46.7% to 75.0%.

After having then defined the chromaticity point having an X value of 0.335 and Y value of 0.335 in the CIE XYZ color system as the standard color (Standard Color 2), specimens having an X value of 0.335 or more and a Y value of 0.335 or more in the same color system were selected as specimens having color depth equal to or higher than that of Standard Color 2. Specimens indicated with a sharp symbol (#) in the "Specimen No." column of Tables 3 to 5 indicate specimens that were selected based on Standard Color 2. Furthermore, only specimens that were the same as the specimens selected based on the aforementioned Standard Color 2 were selected when liquid color having optical absorbance of 0.06 at 455 nm was used for the standard color for each specimen. When only these selected specimens were investigated for sensitivity, sensitivity in the pancreatic cancer (Tis,T1,T2) group was 66.7% (6/9 specimens), sensitivity in the pancreatic cancer (T3) group was 91.0% (20/22 specimens) and sensitivity in the pancreatic cancer (T4) group was 63.6% (7/11 specimens). In addition, the overall sensitivity for 42 specimens selected from among the 49 pancreatic cancer specimens was 78.0%. The case of targeting only those specimens that were selected based on Standard Color 2 resulted in greater improvement of overall sensitivity and sensitivity in the pancreatic cancer (T4) group in comparison with the case of targeting all specimens.

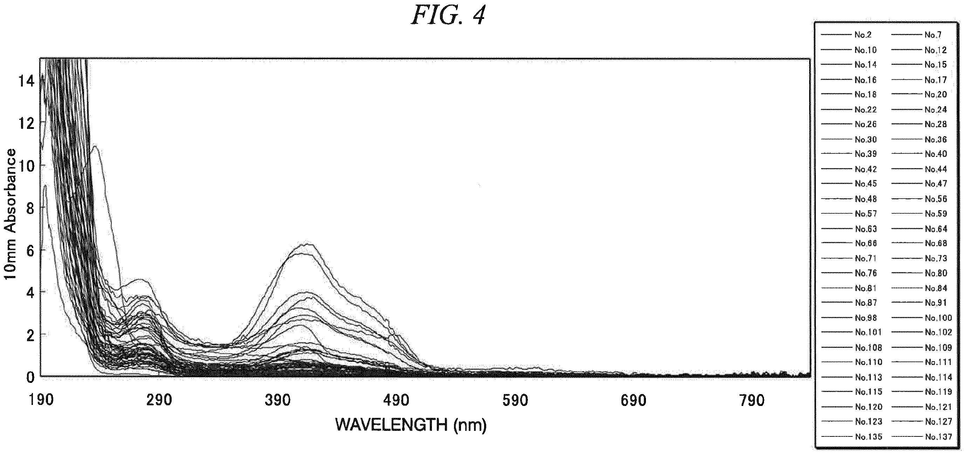

A dilution series obtained by dissolving bilirubin (Bilirubin Conjugate Ditaurate Disodium Salt, Calbiochem Corp.) in water was prepared, the absorption spectrum of each specimen was measured in the same manner as the aforementioned specimens to determine chromaticity, and the resulting chromaticity was plotted on the XY coordinates of a chromaticity diagram of the CIE XYZ color system. In addition, absorption spectrum was similarly measured for water used as a control followed by determination of chromaticity and plotting on the XY coordinates of a chromaticity diagram. The chromaticity point interval on the chromaticity diagram was then calculated between the chromaticity point of each dilution series and the chromaticity point of water. The calculation results are shown in FIG. 4. As a result, chromaticity point intervals between the aqueous bilirubin solution and water on the chromaticity diagram were determined to increase dependent on bilirubin concentration in the case of these aqueous bilirubin solutions.

In addition, the chromaticity point interval between the chromaticity point of water and the chromaticity point of the aforementioned Standard Color 1 (X=0.345, Y=0.349) was 0.0195, and the chromaticity point interval between the chromaticity point of water and the chromaticity point of the aforementioned Standard Color 2 (X=0.335, Y=0.335) was 0.0024. When these chromaticity point intervals are correlated with the calibration curve of FIG. 4, the color depth of Standard Color 1 was determined to be the same as an aqueous bilirubin solution having a concentration of 0.04 mg/mL, while the color depth of Standard Color 2 was determined to be the same as an aqueous bilirubin solution having a concentration of 0.005 mg/mL. On the basis of these results, by selecting duodenal fluid samples using the color of an aqueous bilirubin solution having a concentration of 0.04 mg/mL for Standard Color 1 and using the color of an aqueous bilirubin solution having a concentration of 0.005 ng/mL for Standard Color 2, it is clear that sensitivity in testing for pancreatic disease markers improves and both testing reliability and efficiency are enhanced.

[Reference Example 2]

Duodenal fluid samples were collected transendoscopically from 11 pancreatic cancer cases followed by measuring the CEA concentrations in the samples. CEA concentrations were measured by ELISA using a commercially available kit (IBL International Corp.). The samples were judged to be negative or positive based on the measured CEA concentrations using a cutoff value of 126 ng/mL. Furthermore, specimens M7 and M11 among these 11 cases were the same as specimens M7 and M11 in Reference Example 1 and Example 1.

The results of measuring CEA concentration in each sample are shown in Table 6. As a result, 5 of 11 cases (specimens F67, S136, F9, M7 and M11) were false negatives (cases of pancreatic cancer having CEA concentrations below the threshold value), and sensitivity was 54.5%.

[Example 2]

The absorption spectra were measured for duodenal fluid samples from 9 cases of early pancreatic cancer for which CEA concentration had been measured in Reference Example 2. Each duodenal fluid sample was clearly determined to have an absorption peak wavelength range at 350 nm to 540 nm, and particularly 400 nm to 460 nm.

Moreover, the color depth of each duodenal fluid sample (specimen) was evaluated in the same manner as Example 1. The X and Y values of each specimen along with optical absorbance at 455 nm are shown in Table 6.

[Table 6]

| Pancreatic Cancer Specimens | | | | | | | |
|---|---|---|---|---|---|---|---|
| Specimen No. | CEA concentration (ng/mL) | XYZ color system | | Optical absorbance (455 nm) | Color evaluation | pH | pH evaluation |
| | | X value | Y value | | | | |
| F1 | 415 | 0.406 | 0.434 | 0.316 | ○ | 8.7 | ○ |
| F5 | 130 | 0.473 | 0.482 | 0.042 | ○ | 9.0 | ○ |

(continued)

| Pancreatic Cancer Specimens | | | | | | | |
|---|---|---|---|---|---|---|---|
| Specimen No. | CEA concentration (ng/mL) | XYZ color system | | Optical absorbance (455 nm) | Color evaluation | pH | pH evaluation |
| | | X value | Y value | | | | |
| F55 | 440 | 0.447 | 0.479 | 0.102 | ○ | 9.1 | ○ |
| F57 | 1600 | 0.475 | 0.498 | 0.013 | ○ | 9.3 | ○ |
| F67 | 55 | 0.501 | 0.483 | 0.004 | ○ | 9.0 | ○ |
| S71 | 200 | 0.468 | 0.493 | 0.032 | ○ | 9.0 | ○ |
| S136 | 70 | 0.422 | 0.453 | 0.052 | ○ | 9.3 | ○ |
| S144 | 240 | 0.460 | 0.489 | 0.076 | ○ | 8.4 | ○ |
| F9 | 80 | 0.343 | 0.346 | 0.879 | × | 9.2 | ○ |
| M7 | 54 | 0.385 | 0.409 | 0.731 | ○ | 8.9 | ○ |
| Mil | 30 | 0.374 | 0.388 | 0.497 | ○ | 5.7 | × |

After having defined the chromaticity point having an X value of 0.345 and Y value of 0.349 in the CIE XYZ color system as the standard color (Standard Color 1), specimens having an X value of 0.345 or more and a Y value of 0.349 or more in the same color system were selected as specimens having color depth equal to or higher than that of Standard Color 1. Specimens having color depth equal to or higher than Standard Color 1 were evaluated as being suitable for testing, while specimens having color depth lower than Standard Color 1 were evaluated as being unsuitable for testing. Specimens indicated with a circle (○) in the "Color evaluation" column of Table 6 represent specimens that were evaluated as suitable for testing based on Standard Color 1, while specimens indicated with an X represent specimens that were evaluated as unsuitable for testing. In other words, all specimens other than F9 were evaluated as suitable for testing. Furthermore, only specimens that were the same as the specimens selected based on the aforementioned Standard Color 1 were selected when liquid color having optical absorbance of 0.137 at 455 nm was used for the standard color for each specimen.

Moreover, each specimen was also measured for pH. When using pH 7. 5 for the standard value, specimens having a pH equal to or higher than the standard value were evaluated as suitable for testing, while specimens having a pH lower than the standard value were evaluated as unsuitable for testing. The results for measuring and evaluating pH are shown in Table 6. Specimens indicated with a circle (○) in the "pH evaluation" column of Table 6 represent specimens that were evaluated as suitable for testing based on the standard value (pH 7.5), while specimens indicated with an X represent specimens that were evaluated as unsuitable for testing. As a result, since specimen M11 was evaluated as unsuitable for testing, nine specimens were selected as being suitable for testing together with the results of color evaluation. Specimen M11 was presumed to have been a false negative due to contamination by gastric fluid and a low content of pancreatic fluid.

Sensitivity as determined from CEA concentrations of the selected nine specimens as a result of this screening was 66. 7% (6/9) and sensitivity results improved. On the basis of these results, it was determined that whether or not a duodenal fluid sample is suitable for testing for a pancreatic disease marker (whether or not reliable results can be expected to be obtained when subjected to testing) can be evaluated by using the color and pH of that duodenal fluid sample as indicators, and that a duodenal fluid sample suitable for testing for a pancreatic disease marker can be selected by using color and pH as indicators.

[Example 3]

P-amylase concentrations (enzymatic activity concentrations) were measured for the 11 specimens investigated for color and pH in Example 2.

P-amylase concentrations were measured in accordance with the standard method of the Japan Society of Clinical Chemistry (JSCC). Namely, residual AMY activity, as determined according to an S-AMY inhibition assay using anti-human salivary gland amylase (S-AMY), was measured using a method that complied with the IFCC recommended method.

After defining the standard concentration of the enzymatic activity concentration of P-amylase to be 5000 U/L, specimens having a P-amylase concentration of equal to or higher than the standard concentration were evaluated as suitable for testing, while specimens having a P-amylase concentration lower than the standard concentration were evaluated as unsuitable for testing. The results of measuring the P-amylase concentration of each specimen along with the results of evaluating each specimen based on P-amylase concentration are shown in Table 7 along with the CEA concentrations, color evaluations and pH evaluations of Reference Example 2 and Example 2. Specimens indicated with a circle (○) in the "P-amylase concentration evaluation" column represent specimens that were evaluated as suitable for testing based on the standard value (5000 U/L), while specimens indicated with an X represent specimens that were evaluated as unsuitable for testing.

[Table 7]

| Pancreatic Cancer Specimens | | | | | |
|---|---|---|---|---|---|
| Specimen No. | CEA concentration (ng/mL) | Color evaluation | pH evaluation | P-amylase concentration (U/L) | P-amylase concentration evaluation |
| F1 | 415 | ○ | ○ | 18200 | ○ |
| F5 | 130 | ○ | ○ | 86900 | ○ |
| F55 | 440 | ○ | ○ | 38400 | ○ |
| F57 | 1600 | ○ | ○ | 75300 | ○ |
| F67 | 55 | ○ | ○ | 75400 | ○ |
| S71 | 200 | ○ | ○ | 97900 | ○ |
| S136 | 70 | ○ | ○ | 2600 | x |
| S144 | 240 | ○ | ○ | 475100 | ○ |
| F9 | 80 | × | ○ | 9500 | ○ |
| M7 | 54 | ○ | ○ | 3200 | × |
| M11 | 30 | ○ | × | 46700 | ○ |

Seven specimens were selected based on the color, pH and P-amylase concentration standards of the duodenal fluid samples. Sensitivity as determined from the CEA concentrations of the seven selected specimens was 85.7% (6/7) and sensitivity results improved as a result of that screening. On the basis of these results, duodenal fluid samples suitable for testing for a pancreatic disease marker were determined to be able to be selected by using the color, pH and P-amylase concentration of duodenal fluid samples as indicators. Namely, on the basis of these results, it was determined that whether or not a duodenal fluid sample can be subjected to testing for a pancreatic disease marker can be evaluated by using the color and P-amylase of that duodenal fluid sample as indicators, and that test sensitivity can be improved by selecting duodenal fluid samples based on color and P-amylase concentration.

INDUSTRIAL APPLICABILITY

According to the sample selection method and marker detection method according to the present invention, since the suitability of a duodenal fluid sample can be evaluated and only those samples that are suitable can be subjected to testing for a pancreatic disease marker, these methods can be used in fields relating to analysis of pancreatic disease markers in pancreatic fluid, and in particular, can be used in fields such as clinical laboratory testing for the purpose of diagnosing and treating pancreatic cancer.

**Claims**

1.  A method for selecting a duodenal fluid sample for detecting a pancreatic disease marker, comprising:

   (a1) a step of comparing the color depth of a duodenal fluid sample with a prescribed standard color, and
   (b1) a step of determining that a duodenal fluid sample is subjected to a test for a pancreatic disease marker if the color depth thereof is equal to or higher than the standard color, but that the duodenal fluid sample is not subjected to a test for a pancreatic disease marker if the color depth thereof is lower than the standard color.

2. A method for selecting a duodenal fluid sample for detecting a pancreatic disease marker, comprising:

   (a1) a step of comparing the color depth of a duodenal fluid sample with a prescribed standard color,
   (a2) a step of comparing the pH of the duodenal fluid sample with a prescribed standard value, and
   (b2) a step of determining that a duodenal fluid sample is subjected to a test for a pancreatic disease marker if the color depth thereof is equal to or higher than the standard color and the pH thereof is equal to or higher than the standard value, but that the duodenal fluid sample is not subjected to a test for a pancreatic disease marker if the color depth thereof is lower than the standard color or the pH thereof is lower than the standard value.

3. The method for selecting a duodenal fluid sample for detecting a pancreatic disease marker according to claim 2, wherein the step (a2) is carried out only on a duodenal fluid sample for which the color depth of the duodenal fluid sample is equal to or higher than the standard color as determined in the step (a1).

4. A method for selecting a duodenal fluid sample for detecting a pancreatic disease marker, comprising:

   (a1) a step of comparing the color depth of a duodenal fluid sample with a prescribed standard color,
   (a2) a step of comparing the pH of the duodenal fluid sample with a prescribed standard value,
   (a3) a step of comparing the P-amylase concentration in the duodenal fluid sample with a prescribed standard concentration, and
   (b3) a step of determining that a duodenal fluid sample is subjected to a test for a pancreatic disease marker if the color depth thereof is equal to or higher than the standard color, the pH thereof is equal to or higher than the standard value and the P-amylase concentration thereof is equal to or higher than the standard concentration, but that the duodenal fluid sample is not subjected to a test for a pancreatic disease marker if the color depth thereof is lower than the standard color, the pH thereof is lower than the standard value, or the P-amylase concentration thereof is lower than the standard concentration.

5. The method for selecting a duodenal fluid sample for detecting a pancreatic disease marker according to claim 4, wherein the step (a2) or the step (a3) is carried out only on a duodenal fluid sample that has been determined to be suitable for testing for a pancreatic disease marker in the step (a1).

6. A method for selecting a duodenal fluid sample for detecting a pancreatic disease marker, comprising:

   (a1) a step of comparing the color depth of a duodenal fluid sample with a prescribed standard color,
   (a3) a step of comparing the P-amylase concentration in the duodenal fluid sample with a prescribed standard concentration, and
   (b4) a step of determining that a duodenal fluid sample is subjected to a test for a pancreatic disease marker if the color depth thereof is equal to or higher than the standard color and the P-amylase concentration thereof is equal to or higher than the standard concentration, but that the duodenal fluid sample is not subjected to a test for a pancreatic disease marker if the color depth thereof is lower than the standard color or the P-amylase concentration thereof is lower than the standard concentration.

7. The method for selecting a duodenal fluid sample for detecting a pancreatic disease marker according to claim 6, wherein the step (a3) is carried out only on a duodenal fluid sample that has been determined to be suitable for testing for a pancreatic disease marker in the step (a1).

8. The method for selecting a duodenal fluid sample for detecting a pancreatic disease marker according to any of claims 1 to 7, wherein the standard color is the color of a 0.005 mg/mL aqueous bilirubin solution or a 0.04 mg/mL aqueous bilirubin solution.

9. The method for selecting a duodenal fluid sample for detecting a pancreatic disease marker according to any of claims 1 to 8, wherein the comparing of the color depth of a duodenal fluid sample is carried out visually.

10. The method for selecting a duodenal fluid sample for detecting a pancreatic disease marker according to any of claims 1 to 8, wherein comparison of the color depth of a duodenal fluid sample is carried out based on spectral information of the duodenal fluid sample.

11. The method for selecting a duodenal fluid sample for detecting a pancreatic disease marker according to any of claims 1 to 8, wherein the chromaticity point interval between the duodenal fluid sample and water, and the chromaticity point interval between the standard color and water are compared on a chromaticity diagram of the CIE XYZ color system or CIE L*a*b* color system in the step (a1), and

in the step (b1), (b2), (b3) or (b4), the duodenal fluid sample is determined to be subjected to a test for a pancreatic disease marker if the chromaticity point interval between the duodenal fluid sample and water is equal to or greater than the chromaticity point interval between the standard color and water, but not subjected to a test for a pancreatic disease marker if the chromaticity point interval between the duodenal fluid sample and water is less than the chromaticity point interval between the standard color and water.

12. The method for selecting a duodenal fluid sample for detecting a pancreatic disease marker according to any of claims 1 to 8, wherein the comparing of color depth of a duodenal fluid sample is carried out based on optical absorbance at a prescribed wavelength within the range of 350 nm to 540 nm.

13. The method for selecting a duodenal fluid sample for detecting a pancreatic disease marker according to any of claims 1 to 8, wherein the comparing of color depth of a duodenal fluid sample is carried out based on optical absorbance at a prescribed wavelength within the range of 400 nm to 460 nm.

14. The method for selecting a duodenal fluid sample for detecting a pancreatic disease marker according to any of claims 2 to 5 or any of claims 8 to 13, wherein the prescribed standard value of the pH of the duodenal fluid sample is 7.5 or higher.

15. The method for selecting a duodenal fluid sample for detecting a pancreatic disease marker according to any of claims 4 to 14, wherein the prescribed standard concentration of the P-amylase concentration in the duodenal fluid sample is 5000 U/L or more.

16. A method for detecting a pancreatic disease marker, comprising:

a step of detecting a pancreatic disease marker in a duodenal fluid sample; wherein,
the duodenal fluid sample is selected according to the method for selecting a duodenal fluid sample for detecting a pancreatic disease marker according to any of claims 1 to 15.

17. A method for detecting a pancreatic disease marker, comprising:

(a1) a step of comparing the color depth of a duodenal fluid sample with a prescribed standard color,
(b1) a step of determining that the duodenal fluid sample is subjected to a test for a pancreatic disease marker if the color depth is equal to or higher than the standard color, but that the duodenal fluid sample is not subjected to a test for a pancreatic disease marker if the color depth is lower than the standard color, and
(c1) a step of detecting a pancreatic disease marker in the duodenal fluid sample that has been determined to be subjected to a test for a pancreatic disease marker in the step (b1).

18. The method for detecting a pancreatic disease marker according to claim 16 or claim 17, wherein the pancreatic disease marker is CEA.

*FIG. 1*

```
┌─────────────────────────────────┐
│  PREPARATION OF COLLECTED       │
│  DUODENAL FLUID                 │
└─────────────────────────────────┘
              │
              ▼
┌─────────────────────────────────┐
│  MEASUREMENT OF OPTICAL         │
│  SPECTRUM OF DUODENAL FLUID     │
└─────────────────────────────────┘
              │
              ▼
        DETERMINATION OF
    WHETHER OR NOT SAMPLE IS              TESTING
      SUBJECTED TO TESTING               NOT PERMITTED
        USING SPECTRAL
         INFORMATION
              │
     TESTING PERMITTED
              │
              ▼
┌─────────────────────────────────┐
│  MEASUREMENT OF PANCREATIC      │
│  DISEASE MARKER IN DUODENAL FLUID│
└─────────────────────────────────┘

NEGATIVE                 POSITIVE

┌──────────┐      ┌──────────────┐      ┌──────────────┐
│  NORMAL  │      │   DETAILED   │      │ OTHER TESTS  │
│          │      │ EXAMINATION  │      │  PERFORMED   │
└──────────┘      └──────────────┘      └──────────────┘
```

## FIG. 2

PREPARATION OF COLLECTED
DUODENAL FLUID

↓

MEASUREMENT OF OPTICAL
SPECTRUM OF DUODENAL FLUID

↓

COMPARISON OF
COLOR WITH STANDARD COLOR
USING SPECTRAL
INFORMATION

LIGHTER THAN
STANDARD COLOR →

EQUAL TO OR DARKER THAN
STANDARD COLOR
↓

COMPARISON OF PH WITH
STANDARD VALUE

LOWER THAN
STANDARD VALUE →

EQUAL TO OR HIGHER THAN
STANDARD VALUE
↓

COMPARISON OF
P-AMYLASE CONCENTRATION
WITH STANDARD
CONCENTRATION

LOWER THAN
STANDARD
CONCENTRATION →

EQUAL TO OR HIGHER THAN
STANDARD CONCENTRATION
↓

MEASUREMENT OF PANCREATIC
DISEASE MARKER IN DUODENAL FLUID

NEGATIVE          POSITIVE

NORMAL     DETAILED
EXAMINATION     OTHER TESTS
PERFORMED

## FIG. 3

```
┌─────────────────────────────────┐
│   PREPARATION OF COLLECTED       │
│        DUODENAL FLUID            │
└─────────────────────────────────┘
                │
                ▼
┌─────────────────────────────────┐
│   MEASUREMENT OF OPTICAL         │
│   SPECTRUM OF DUODENAL FLUID     │
└─────────────────────────────────┘
                │
                ▼
        ◇ COMPARISON OF                LIGHTER THAN
          COLOR WITH STANDARD COLOR    STANDARD COLOR
          USING SPECTRAL ──────────────────────────────┐
          INFORMATION                                   │
                │                                        │
     EQUAL TO OR DARKER THAN                             │
     STANDARD COLOR                                      │
                │                                        │
                ▼                                        │
        ◇ COMPARISON OF                LOWER THAN        │
          P-AMYLASE CONCENTRATION      STANDARD          │
          WITH STANDARD ──────────────CONCENTRATION      │
          CONCENTRATION                         │        │
                │                               │        │
     EQUAL TO OR HIGHER THAN                    │        │
     STANDARD CONCENTRATION                     │        │
                │                               │        │
                ▼                               │        │
        ◇ COMPARISON OF PH WITH        LOWER THAN        │
          STANDARD VALUE ──────────── STANDARD VALUE     │
                │                               │        │
     EQUAL TO OR HIGHER THAN                    │        │
     STANDARD VALUE                             │        │
                │                               │        │
                ▼                               │        │
┌─────────────────────────────────┐            │        │
│   MEASUREMENT OF PANCREATIC      │            │        │
│   DISEASE MARKER IN DUODENAL     │            │        │
│           FLUID                  │            │        │
└─────────────────────────────────┘            │        │
        │                │                      │        │
   NEGATIVE          POSITIVE                   │        │
        │                │                      │        │
        ▼                ▼                      ▼        ▼
  ┌──────────┐    ┌──────────────┐        ┌──────────────┐
  │  NORMAL  │    │   DETAILED   │        │ OTHER TESTS  │
  │          │    │ EXAMINATION  │        │  PERFORMED   │
  └──────────┘    └──────────────┘        └──────────────┘
```

*FIG. 4*

| | |
|---|---|
| No.2 | No.7 |
| No.10 | No.12 |
| No.14 | No.15 |
| No.16 | No.17 |
| No.18 | No.20 |
| No.22 | No.24 |
| No.26 | No.28 |
| No.30 | No.36 |
| No.39 | No.40 |
| No.42 | No.44 |
| No.45 | No.47 |
| No.48 | No.56 |
| No.57 | No.59 |
| No.63 | No.64 |
| No.66 | No.68 |
| No.71 | No.73 |
| No.76 | No.80 |
| No.81 | No.84 |
| No.87 | No.91 |
| No.98 | No.100 |
| No.101 | No.102 |
| No.108 | No.109 |
| No.110 | No.111 |
| No.113 | No.114 |
| No.115 | No.119 |
| No.120 | No.121 |
| No.123 | No.127 |
| No.135 | No.137 |

*FIG. 5*

*FIG. 6*

## INTERNATIONAL SEARCH REPORT

| International application No. |
|---|
| PCT/JP2014/060675 |

**A. CLASSIFICATION OF SUBJECT MATTER**

*G01N33/72*(2006.01)i, *G01J3/42*(2006.01)i, *G01J3/46*(2006.01)i, *G01N33/50* (2006.01)i, *G01N33/52*(2006.01)i, *G01N33/68*(2006.01)i

According to International Patent Classification (IPC) or to both national classification and IPC

**B. FIELDS SEARCHED**

Minimum documentation searched (classification system followed by classification symbols)
G01N33/72, G01J3/42, G01J3/46, G01N33/50, G01N33/52, G01N33/68

Documentation searched other than minimum documentation to the extent that such documents are included in the fields searched
Jitsuyo Shinan Koho        1922–1996   Jitsuyo Shinan Toroku Koho   1996–2014
Kokai Jitsuyo Shinan Koho   1971–2014   Toroku Jitsuyo Shinan Koho   1994–2014

Electronic data base consulted during the international search (name of data base and, where practicable, search terms used)
CAplus/MEDLINE/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| A | Microfilm of the specification and drawings annexed to the request of Japanese Utility Model Application No. 216/1985(Laid-open No. 118309/1986) (Olympus Optical Co., Ltd.), 25 July 1986 (25.07.1986), page 7, lines 7 to 17 & US 4724836 A | 1-18 |
| A | WO 2002/020061 A1 (Tokyo Gas Co., Ltd.), 14 March 2002 (14.03.2002), Background Art & US 2003/0228647 A1    & US 2006/0292660 A1 & US 2007/0086945 A1    & EP 1316320 A1 | 1-18 |

|☒| Further documents are listed in the continuation of Box C. |☐| See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 July, 2014 (02.07.14) | 15 July, 2014 (15.07.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2014/060675

C (Continuation).    DOCUMENTS CONSIDERED TO BE RELEVANT

| Category* | Citation of document, with indication, where appropriate, of the relevant passages | Relevant to claim No. |
|---|---|---|
| A | WO 2013/031643 A1 (Olympus Corp.), 07 March 2013 (07.03.2013), claims (Family: none) | 1-18 |
| A | WO 2013/038981 A1 (Olympus Corp.), 21 March 2013 (21.03.2013), claims (Family: none) | 1-18 |

Form PCT/ISA/210 (continuation of second sheet) (July 2009)

**EP 3 018 480 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013138848 A
- WO 2013038981 A
- JP 2006308576 A

**Non-patent literature cited in the description**

- **ROLNY et al.** *Scandinavian Journal of Gastroenterology,* 1977, vol. 12, 759-763
- *Pancreas,* 1994, vol. 9 (6), 741-747
- *Pancreas,* 2002, vol. 24 (54), 344-347